Europäisches Patentamt

European Patent Office

Office européen des brevets

(11) Veröffentlichungsnummer : **0 146 718**
**B1**

(12) **EUROPÄISCHE PATENTSCHRIFT**

(45) Veröffentlichungstag der Patentschrift :
27.05.87

(51) Int. Cl.⁴ : **A 61 B   6/00**, A 61 B   6/04

(21) Anmeldenummer : **84112772.3**

(22) Anmeldetag : **23.10.84**

(54) **Röntgendiagnostikgerät mit einem Kompressionswagen.**

(30) Priorität : **08.11.83 DE 8332063 U**

(43) Veröffentlichungstag der Anmeldung :
**03.07.85 Patentblatt 85/27**

(45) Bekanntmachung des Hinweises auf die Patenterteilung : **27.05.87 Patentblatt 87/22**

(84) Benannte Vertragsstaaten :
**DE FR**

(56) Entgegenhaltungen :
**BE-A-   811 497**
**DE-A- 3 140 225**
**FR-A- 2 417 128**
**US-A- 3 991 316**

(73) Patentinhaber : **Siemens Aktiengesellschaft Berlin und München**
**Wittelsbacherplatz 2**
**D-8000 München 2 (DE)**

(72) Erfinder : **Summ, Herbert**
**Erlangerstrasse 8**
**D-8531 Wilhelmsdorf (DE)**

EP 0 146 718 B1

## Beschreibung

Die Erfindung betrifft ein Röntgendiagnostikgerät mit einem durch einen Elektromotor verstellbaren Kompressionswagen, der Halterungsmittel für einen Kompressionstubus aufweist, und mit einer Aufnahmeplatte für das Aufnahmeobjekt.

Es sind Röntgendiagnostikgeräte dieser Art bekannt (US-A-3 991 316, BE-A-811 497), die zur Anfertigung von Aufnahmen der weiblichen Brust dienen. Die Stellung des Kompressionstubus in bezug auf den Kompressionswagen ist dabei derart festgelegt, daß aufgrund des zur Verfügung stehenden Weges für den Kompressionswagen einerseits eine einwandfreie Lagerung des Aufnahmeobjektes auf einer Aufnahmeplatte und andererseits eine sichere Kompression möglich sind.

Der Erfindung liegt die Aufgabe zugrunde, ein Röntgendiagnostikgerät der eingangs genannten Art so auszubilden, daß Vergrößerungsaufnahmen möglich sind, bei denen das Aufnahmeobjekt vergrößert auf einem Röntgenfilm abgebildet wird, wobei eine sichere Kompression sowohl bei normalen als auch bei Vergrößerungsaufnahmen gewährleistet ist.

Diese Aufgabe ist erfindungsgemäß dadurch gelöst, daß am Halter (3) Halterungsmittel für eine im Vergleich zur Aufnahmeplatte fokusnähere Auflageplatte und am Kompressionswagen zwei Haltevorrichtungen für den Kompressionstubus vorgesehen sind, von denen die eine der Aufnahmeplatte mit der Röntgenfilmkassette und die andere der fokusnäheren Auflageplatte zugeordnet ist. Bei dem erfindungsgemäßen Röntgendiagnostikgerät stehen für den Kompressionstubus bei gegebenem Verstellweg für den Kompressionswagen zwei Verstellbereiche zur Verfügung, von denen der eine der Aufnahmeplatte mit der Röntgenfilmkassette und der andere bei Vergrößerungsaufnahmen der fokusnäheren Auflageplatte zugeordnet ist.

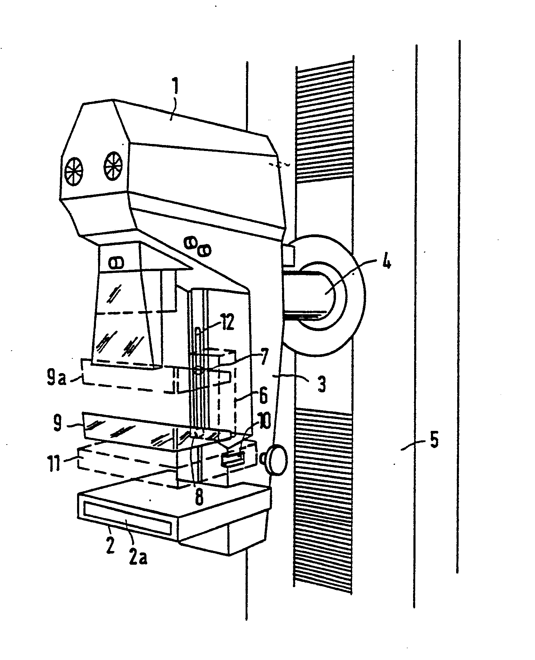

Die Erfindung ist nachfolgend anhand eines in der Zeichnung dargestellten Ausführungsbeispieles näher erläutert.

In der Zeichnung sind ein Gehäuse 1 für eine Röntgenröhre und eine Aufnahmeplatte 2 zur Anfertigung üblicher Mammographien dargestellt, in die eine Röntgenfilmkassette 2a einschiebbar ist. Das Gehäuse 1 und die Aufnahmeplatte 2 sind über einen Halter 3 miteinander verbunden, der eine horizontale Achse 4 aufweist, mit der er an einem Stativ 5 höhenverstellbar gelagert ist. Die Einheit 1, 2 ist ferner um die Achse 4 drehbar.

Im Halter 3 ist ein motorisch verstellbarer Kompressionswagen 6 vorgesehen, der zwei Öffnungen 7, 8 aufweist. Ein Kompressionstubus 9 steckt mit einem Ansatz bei dem Beispiel in der Öffnung 8. Wird das Aufnahmeobjekt auf die Aufnahmeplatte 2 gelegt, so kann es durch den Kompressionstubus 9 durch motorische Verstellung des Kompressionswagens 6 nach unten komprimiert werden.

Für die Anfertigung von Vergrößerungsaufnahmen kann am Halter 3 eine Auflageplatte 11 befestigt werden, die fokusnäher als die Aufnahmeplatte 2 mit der Röntgenfilmkassette liegt. Damit bei demselben Verstellweg des Kompressionswagens 3 auch in diesem Fall eine sichere Kompression des Aufnahmeobjektes möglich ist, wird in diesem Fall der Kompressionstubus 9 in seiner gestrichelt gezeichneten Stellung, in der er mit 9a bezeichnet ist, mit seinem Ansatz in die Öffnung 7 des Kompressionswagens 3 eingeschoben. Er liegt dabei in jedem Fall über der Auflageplatte 11, die mit Hilfe einer Halterungsvorrichtung 10 am Halter 3 befestigt wird. Der Kompressionstubus 9 ist mit seinem Stift, mit dem er in die Öffnungen 7, 8 eingeschoben wird, in einem Schlitz 12 des Halters 3 längsverschiebbar.

## Patentanspruch

Röntgendiagnostikgerät mit einem Halter (3) für die Röntgenröhre und einer Aufnahmeplatte (2) sowie mit einem in Richtung auf die Aufnahmeplatte (2) motorisch verstellbaren Kompressionswagen (6), der Halterungsmittel (7, 8) für einen Kompressionstubus (9) aufweist, dadurch gekennzeichnet, daß am Halter (3) Halterungsmittel (10) für eine im Vergleich zur Aufnahmeplatte (2), fokusnähere Auflageplatte (11) und am Kompressionswagen (6) zwei Haltevorrichtungen (7, 8) für den Kompressionstubus (9) vorgesehen sind, von denen die eine (8) der Aufnahmeplatte (2) mit der Röntgenfilmkassette und die andere (7) der fokusnäheren Auflageplatte (11) zugeordnet ist.

## Claim

X-ray diagnostic apparatus comprising a holder (3) for the X-ray tube and a photograph plate (2) and a compression device (6) which is adjustable by means of a motor in the direction of the photograph plate (2) and has holding means (7, 8) for a compression tube (9), characterised in that on the holder (3) there are arranged holding means (10) for a supporting plate (11) which is closer to the focal point as compared with the photograph plate (2), and at the compression device, two holding devices (7, 8) for the compression tube (9), one (8) of which is assigned to the photograph plate (2) with the X-ray film cassette and the other (7) of which is assigned to the contact plate (11) which is closer to the focal point.

## Revendication

Appareil de radiodiagnostic comprenant un chariot de compression (6) comportant un support (3) pour le tube à rayons X et une plaque d'enregistrement (2) ainsi qu'un chariot de compression (6), qui est déplaçable au moyen d'un moteur en direction de la plaque d'enregistrement (2) et qui comporte des moyens (7, 8) de maintien pour un compresseur (9), caractérisé par le fait que sur le support (3), il est prévu des moyens de maintien (10) pour une plaque de support (11) plus proche du foyer que la plaque d'enregistrement (2), et, sur le chariot de compression (6), deux dispositifs de maintien (7, 8) pour le compresseur (9), dont l'un (8) est associé à la plaque d'enregistrement (2) comportant la cassette à film radiographique et dont l'autre (7) est associé à la plaque de support (11) qui est plus proche du foyer.